(19) Europäisches Patentamt
European Patent Office
Office européen des brevets

(11) **EP 4 265 668 A1**

(12) **EUROPEAN PATENT APPLICATION**
published in accordance with Art. 153(4) EPC

(43) Date of publication:
**25.10.2023 Bulletin 2023/43**

(21) Application number: **21906729.5**

(22) Date of filing: **17.12.2021**

(51) International Patent Classification (IPC):
***C08G 65/40*** *(2006.01)* ***C09K 11/06*** *(2006.01)*
***A01P 1/00*** *(2006.01)* ***A01N 65/00*** *(2009.01)*
***A01P 3/00*** *(2006.01)*

(52) Cooperative Patent Classification (CPC):
**A01N 65/00; A01P 1/00; A01P 3/00; C08G 65/40; C09K 11/06**

(86) International application number:
**PCT/JP2021/046806**

(87) International publication number:
**WO 2022/131371 (23.06.2022 Gazette 2022/25)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.12.2020 JP 2020210626**

(71) Applicants:
- **TOPPAN INC.**
  **Tokyo 110-0016 (JP)**
- **National Institute for Materials Science**
  **Tsukuba-shi, Ibaraki 305-0047 (JP)**

(72) Inventors:
- **MATSUBARA Ryohei**
  **Tokyo 110-0016 (JP)**
- **YAMAMOTO Takako**
  **Tokyo 110-0016 (JP)**
- **HASEGAWA Jun**
  **Tokyo 110-0016 (JP)**
- **NAITO Masanobu**
  **Tsukuba-shi, Ibaraki 305-0047 (JP)**
- **FUJITA Takehiro**
  **Tsukuba-shi, Ibaraki 305-0047 (JP)**

(74) Representative: **Hoffmann Eitle**
**Patent- und Rechtsanwälte PartmbB**
**Arabellastraße 30**
**81925 München (DE)**

(54) **POLYPHENOL DERIVATIVE AND POLYMER MATERIAL**

(57) To provide a material that can be easily ascertained whether antibacterial or antiviral function is imparted. In the polyphenol derivative, hydrogen atoms of some hydroxy groups or some hydrogen atoms of aromatic rings of a polyphenol are substituted by a luminescent group. In the polyphenol derivative, hydrogen atoms of some hydroxy groups or some hydrogen atoms of aromatic rings of a polyphenol may be substituted by a chain hydrocarbon group. The polyphenol derivative may be network polymerized into a polymer material.

FIG. 1

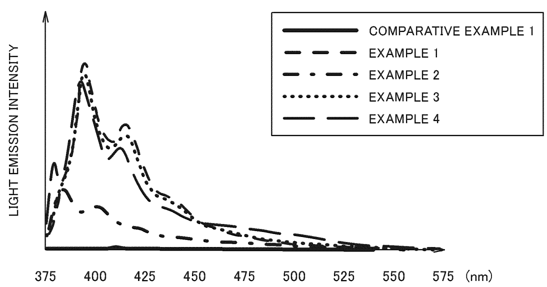


EP 4 265 668 A1

## Description

### Technical Field

The present disclosure relates to a polyphenol derivative and a polymer material.

### Background Art

In recent years, an antibacterial or antiviral need for public space or residential space has grown due to an increasing population of elderly people with low resistance as well as an increasing number of what are called private accommodation services or car-sharing services used by an unspecified number of users. Reducing workload for maintenance and cleaning operations has also been needed due to the decrease in the working population.

To address these needs, antibacterial agents, antiviral agents, or similar agents have been used to impart antibacterial or antiviral function to the surface of interior parts of buildings, transport vehicles, and the like (see PTL 1).

### Citation List

### Patent Literature

PTL 1: WO 2015/198890

### Summary of Invention

### Technical Problem

Conventional antibacterial or antiviral agents, however, contain, as an antibacterial or antiviral substance, a metal (specifically silver), which may change color or increases cost. In addition, even when such an antibacterial or antiviral agent is applied to an interior part, it has been difficult to ascertain whether antibacterial or antiviral function is actually imparted.

The present disclosure is therefore intended to provide a polyphenol derivative and a polymer material to be used as an antibacterial or antiviral agent that can be easily ascertained whether antibacterial or antiviral function is imparted.

### Solution to Problem

To solve the above problems, a polyphenol derivative pertaining to an aspect of the present disclosure is characterized in that a hydrogen atom of some hydroxy groups of a polyphenol or some hydrogen atoms of an aromatic ring of a polyphenol are substituted by a luminescent group.

A polymer material pertaining to an aspect of the present disclosure is characterized by including a polyphenol derivative in which a hydrogen atom of some hydroxy groups of a polyphenol or some hydrogen atoms of an aromatic ring of a polyphenol are substituted by a luminescent group, and the polyphenol is network polymerized.

### Advantageous Effects of Invention

According to the present disclosure, a polyphenol derivative and a polymer material that is usable as an antibacterial or antiviral agent and can be easily ascertained whether antibacterial or antiviral function is imparted can be provided.

### Brief Description of Drawings

FIG. 1 is a graph illustrating the results of fluorescence spectrum measurement in examples in the present disclosure.

### Description of Embodiments

Embodiments of the present disclosure will now be described. The embodiments described below are however merely illustrative, and are not intended to exclude various modifications or technology not clearly described below. The present disclosure may be implemented with various modifications (for example, by combining embodiments) without

departing from the scope of the disclosure. In the following drawings, identical or similar parts are indicated by an identical or similar sign.

1. First embodiment (polyphenol derivative)

A polyphenol derivative pertaining to a first embodiment will be described. The polyphenol derivative pertaining to the present embodiment is used as an antibacterial or antiviral agent for imparting antibacterial or antiviral function to the surface of an intended parts and enables visual identification of the presence of antibacterial or antiviral function.

<Structure of polyphenol derivative>

The polyphenol derivative pertaining to the present embodiment is a composition having antibacterial or antiviral performance. In the polyphenol derivative pertaining to the present embodiment, hydrogen atoms of some hydroxy groups or some hydrogen atoms of aromatic rings of a polyphenol are substituted by a luminescent group. Accordingly, the polyphenol derivative has light emitting properties due to the luminescent group bonded to the polyphenol. In other words, the polyphenol derivative pertaining to the present embodiment emits light from a luminescent group bonded to the polyphenol where the polyphenol having antibacterial or antiviral performance exists. Hence, in a region capable of exerting antibacterial or antiviral performance due to the presence of the polyphenol derivative functioning as an antibacterial or antiviral agent, for example, ultraviolet irradiation allows the polyphenol derivative to emit visible light, and the antibacterial or antiviral effect of the polyphenol derivative can be visually identified.

In the description, "antibacterial or antiviral performance" means having at least one performance of sterilization/virus killing (microorganism killing), bacteriostasis/virustasis (microorganism growth suppressing), bacterial killing/virus killing, disinfection, microbe growth control/virus growth control, microbe removal/virus removal, antisepsis, mold prevention, and similar performances against microorganisms including bacteria, fungi, and viruses.

Examples of the polyphenol included in the polyphenol derivative include tannic acids, lignins, catechin, and chlorogenic acid. Of them, the polyphenol is preferably a tannic acid. In other words, the polyphenol derivative is preferably a tannic acid derivative in which hydrogen atoms of some hydroxy groups or some hydrogen atoms of aromatic rings of a tannic acid are substituted by a luminescent group.

The tannic acid is a general term of a plant component that is hydrolyzed into polyhydric phenols. As the tannic acid used in the polyphenol derivative pertaining to the present embodiment, either a hydrolyzable tannic acid in which gallic acid or ellagic acid is ester bonded to a sugar such as glucose and is easily hydrolyzed with an enzyme and a condensed tannic acid in which a compound having a flavanol skeleton is polymerized may be used. These tannic acids may be used singly or as a mixture. Of them, a hydrolyzable tannic acid is preferably used, and, for example, a composition mainly containing a tannic acid represented by Chemical Formula (1) is preferably derivatized.

[Chemical Formula 1]

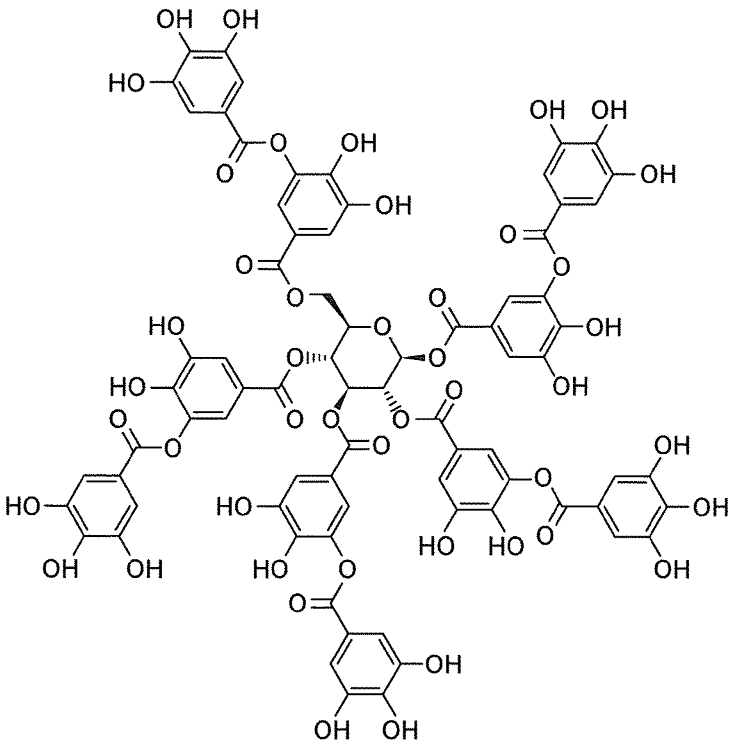

· · ·(1)

As shown in Chemical Formula (1), a polyphenol such as a tannic acid has a plurality of hydroxy groups at terminals. As described above, in the polyphenol derivative pertaining to the present embodiment, hydrogen atoms of some hydroxy groups or some hydrogen atoms of aromatic rings of a polyphenol are substituted by a luminescent group described later. For example, in a tannic acid derivative as an example of the polyphenol derivative pertaining to the present embodiment, hydrogen atoms of some hydroxy groups or some hydrogen atoms of aromatic rings of a tannic acid are substituted by a luminescent group described later. Accordingly, the luminescent group bonded to a polyphenol emits light where the polyphenol exists, and this enables visual identification of the antibacterial or antiviral effect arising from the polyphenol. In the description, "visual identification" means that visible light is identified directly by the human eye (i.e., visually) and that invisible light detected by an invisible light detector or the like is visually identified as information displayed on a display of the apparatus.

In the polyphenol derivative, either hydrogen atoms of hydroxy groups or hydrogen atoms of aromatic rings of a polyphenol may be substituted by a luminescent group. When hydrogen atoms of hydroxy groups are substituted in a polyphenol having n pieces of hydroxy groups, the number of hydroxy groups substituted with a luminescent group is preferably not less than 1 and not more than n - 1 in the polyphenol, and only one hydroxy group is more preferably substituted with a luminescent group. When hydrogen atoms of aromatic rings are substituted in a polyphenol having m pieces of hydrogen atoms on aromatic rings, the number of hydrogen atoms substituted by a luminescent group on the aromatic rings is preferably not less than 1 and not more than m, and only one hydrogen atom of an aromatic ring is more preferably substituted by a luminescent group.

Hence, in the polyphenol derivative, hydrogen atoms of hydroxy groups or aromatic rings of a polyphenol are preferably substituted by a luminescent group such that the number x of luminescent groups is not less than 1 and not more than a value 1 less than the total of the number n of hydrogen atoms of hydroxy groups and the number m of hydrogen atoms of aromatic rings of the polyphenol and is more preferably 1. In other words, the polyphenol derivative is preferably substituted with a luminescent group such that Equation (1) is satisfied.

1 ≤ the number x of luminescent groups ≤ the number n of hydroxy groups - 1 + the number m of hydrogen atoms of aromatic rings ···(1)

(In the equation, x, n, and m are each a positive integer)

This enables visual identification of the antibacterial or antiviral effect arising from a polyphenol. A polyphenol modified with at least one luminescent group has luminescent function, and thus any one hydrogen of hydroxy groups and aromatic rings is preferably substituted by a luminescent group in a polyphenol because the polyphenol obtains luminescent performance while exerting antibacterial or antiviral effect to the maximum extent.

For example, the total number of hydrogen atoms of hydroxy groups and the total number of hydrogen atoms of aromatic rings of a tannic acid vary with the type of used tannic acid. For example, for the tannic acid of Chemical Formula (1), the number of hydroxy groups (the total number of hydrogen atoms) is 25, and the total number of hydrogen atoms of aromatic rings is 20. In the tannic acid represented by Chemical Formula (1), the number x of luminescent groups is preferably 1 or more and 44 or less, and one hydrogen atom is more preferably substituted by a luminescent group.

As described above, when the number of luminescent group-substituted hydrogen atoms of hydroxy groups and aromatic rings in a polyphenol is not less than 1 and not more than (n - 1) + m, the antibacterial or antiviral effect arising from the polyphenol can be visually identified.

In the polyphenol derivative, some hydrogen atoms of hydroxy groups and aromatic rings of a polyphenol, or at least one hydrogen atom of hydroxy groups and aromatic rings of a polyphenol, may be substituted by a chain hydrocarbon group described later. A polyphenol derivative in which some hydrogen atoms of hydroxy groups and aromatic rings of a polyphenol are substituted by a chain hydrocarbon group described later has higher affinity with organic solvents. Hence, for some applications, some hydrogen atoms of hydroxy groups and aromatic rings of a polyphenol are preferably substituted by a chain hydrocarbon group described later.

In a polyphenol derivative in which the number of hydrogen atoms of hydroxy groups of a polyphenol is n, and the number of hydrogen atoms of aromatic rings of the polyphenol is m, hydrogen atoms of hydroxy groups or aromatic rings of the polyphenol are preferably substituted by a chain hydrocarbon group such that the total (x + y) of the number (x) of luminescent groups and the number (y) of chain hydrocarbon groups is not more than a value 1 less than the total (n + m) of the number (n) of hydrogen atoms of hydroxy groups and the number (m) of hydrogen atoms of aromatic rings of the polyphenol. In other words, the polyphenol derivative is preferably substituted with a chain hydrocarbon group such that Equation (2) is satisfied.

The number x of luminescent groups + the number y of chain hydrocarbon groups ≤ the number n of hydroxy group - 1 + the number m of hydrogen atoms of aromatic rings ···(2)

(In the equation, x, y, n, and m are each a positive integer)

In the tannic acid represented by Chemical Formula (1), the number of hydroxy groups (the total number of hydrogen atoms) is 25, and the total number of hydrogen atoms of aromatic rings is 20. Hence, when the number of luminescent groups is 1 in the tannic acid represented by Chemical Formula (1), 1 or more and 43 or less hydrogen atoms of hydroxy groups and hydrogen atoms of aromatic rings are preferably substituted by a chain hydrocarbon group.

When a larger number of hydrogen atoms of hydroxy groups and a larger number of hydrogen atoms of aromatic rings are substituted by a chain hydrocarbon group, a higher affinity with organic solvents is achieved. In the case, a smaller number of hydrogen atoms of hydroxy groups and hydrogen atoms of aromatic rings are substituted by a luminescent group, and the luminescent performance deteriorates. The number of substituents is thus preferably determined according to an intended luminescent performance or the material of a face onto which the polyphenol derivative is to be applied. For example, when a polyphenol derivative having antibacterial or antiviral function is applied to a polar substrate such as a metal substrate and a glass substrate, 80% or less of the total of the number of hydrogen atoms of hydroxy groups and the number of hydrogen atoms of aromatic rings is preferably substituted, and 60% or less is more preferably substituted. For example, for the tannic acid represented by Chemical Formula (1), 36 or less hydrogen atoms of hydroxy groups and hydrogen atoms of aromatic rings are preferably substituted by a chain hydrocarbon group, and 27 or less hydrogen atoms of hydroxy groups and hydrogen atoms of aromatic rings are more preferably substituted by a chain hydrocarbon group.

In the polyphenol derivative, either hydrogen atoms of hydroxy groups or hydrogen atoms of aromatic rings of a polyphenol are preferably substituted by a luminescent group, but both hydrogen atoms of hydroxy groups and hydrogen atoms of aromatic rings may be substituted by a luminescent group.

The structure of such a polyphenol derivative as described above can be identified, for example, on the basis

of an NMR spectrum obtained by using a nuclear magnetic resonance (NMR) apparatus.

(Luminescent group)

As the luminescent group, any group that emits light by any means and enables visual identification of the presence of a polyphenol having antibacterial or antiviral performance can be used.

Light emission from a luminescent group may be, for example, light emission by luminescence such as photoluminescence. In particular, a luminescent group emitting light by photoluminescence is preferred because antibacterial or antiviral performance is easily ascertained.

The light emitted from the luminescent group may be visible light or invisible light, but is preferably visible light because antibacterial or antiviral performance is more easily ascertained.

The means to allow the polyphenol derivative to emit light may be appropriately selected according to a luminescent group. When the polyphenol derivative is a photoluminescence material, light having a certain wavelength, such as ultraviolet light, may be applied. When the polyphenol derivative is a chemical luminescence material, a certain liquid may be applied, or a gas may be sprayed as the means to allow the polyphenol derivative to emit light.

When the polyphenol derivative emits invisible light, an invisible light detector can be used as the means to identify light emitted from the polyphenol derivative.

As described above, as the luminescent group, a functional group capable of emitting visible light by photoluminescence is preferably used. More specifically, a luminescent group that emits fluorescence by ultraviolet light in a wavelength region of, for example, 200 nm or more and 400 nm or less, preferably 300 nm or more and 380 nm or less, is preferred, and a luminescent group that emits fluorescence by black light (a wavelength of 365 nm), which is easily handled, is particularly preferred.

Examples of such a luminescent group include a pyrene group, an anthracene group, a phenanthrene group, a benzoxazole group, a flavone group, a carbazole group, and a coumarin group.

Of them, the pyrene group is preferably a luminescent group having at least one skeleton selected from the group consisting of a 4-(1-pyrene)-butyric acid skeleton, a 1-pyrenebutyric acid skeleton, and a 1-(methyl)pyrene skeleton. The luminescent group having such a skeleton is, for example, a functional group derived from 4-(1-pyrene)-butyryl chloride represented by Chemical Formula (2), 1-pyrenebutyric acid represented by Chemical Formula (3), or 1-(bromomethyl)pyrene represented by Chemical Formula (4).

[Chemical Formula 2]

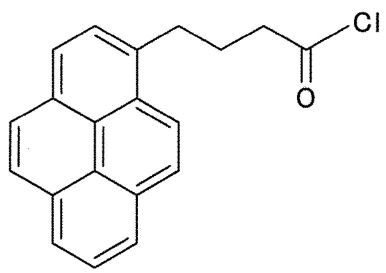

・・・(2)

[Chemical Formula 3]

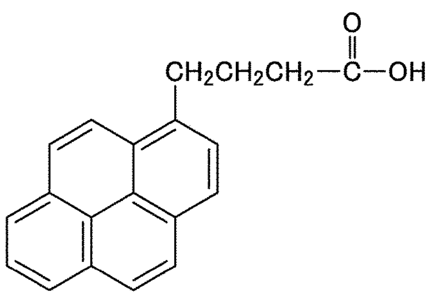

・・・(3)

[Chemical Formula 4]

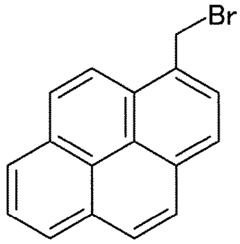

・・・(4)

(Chain hydrocarbon group)

Examples of the chain hydrocarbon group include linear or branched alkyl groups, alkenyl groups, and alkynyl groups, and alkyl groups are specifically preferred. Such a chain hydrocarbon group is bonded through a bond containing an oxygen atom derived from a hydroxy group to a polyphenol skeleton. Examples of the bond containing an oxygen atom include an ether bond, an ester bond, and a urethane bond. A functional group other than the chain hydrocarbon group can be used to chemically modify a polyphenol when having high affinity with organic solvents and having film forming performance on a face to which a polyphenol derivative is applied.

The chain hydrocarbon group preferably has 1 or more and 18 or less carbon atoms, more preferably 4 or more and 18 or less carbon atoms, and even more preferably 6 or more and 16 or less carbon atoms. Specific examples of the chain hydrocarbon group having 1 or more and 18 or less carbon atoms include a methyl group, an ethyl group, a butyl group, a hexyl group, a heptyl group, an octyl group, an isooctyl group, a nonyl group, an isononyl group, a decyl group, an undecyl group, a dodecyl group, a hexadecyl group, a propylene group, a hexylene group, a hexadecenyl group, and an octadecenyl group.

<Method for producing polyphenol derivative>

The polyphenol derivative is produced through a step of chemically modifying a polyphenol with a luminescent group. The polyphenol derivative may also be produced through an additional step of chemically modifying a polyphenol with a chain hydrocarbon group. The step of chemically modifying a polyphenol with a luminescent group and the step of chemically modifying a polyphenol with a chain hydrocarbon group may be performed in any order or may be performed at the same time.

In the step of chemically modifying a polyphenol with a luminescent group, for example, esterification is performed. Specifically, a polyphenol is reacted with a luminescent compound having a carboxyl group in a solvent such as dimethylformamide and dimethyl sulfoxide in the presence of an acidic catalyst, and the polyphenol can be chemically modified with the luminescent group.

As the luminescent compound having a carboxyl group, 1-pyrenecarboxylic acid, 9-anthracenecarboxylic acid, 9-phenanthrenecarboxylic acid, or the like can be used.

As the acidic catalyst, a catalyst donating $H^+$, such as concentrated sulfuric acid, phosphoric acid, and toluenesulfonic acid, can be used.

Esterification is preferably performed by reaction in an environment of 20°C or more and 50°C or less for about 24 hours. By changing the molar ratio of a luminescent compound having a carboxyl group to a polyphenol, the proportion of the luminescent group introduced to the polyphenol can be adjusted at an intended value.

In the step of chemically modifying a polyphenol with a luminescent group, for example, Williamson ether synthesis, an alkylation reaction, may be performed. Specifically, a polyphenol is reacted with a luminescent compound having a halogenated alkyl group in a solvent such as tetrahydrofuran and dimethyl sulfoxide in the presence of a basic catalyst, and the polyphenol can be chemically modified with the luminescent group.

As the luminescent compound having a halogenated alkyl group, 1-bromomethylpyrene, 9-bromomethylanthracene, 4-bromomethyl-7-diethylaminocoumarin, or the like can be used.

As the basic catalyst, one or two or more catalysts selected from the group of MH, $M_2CO_3$, and M (M: alkali metal) can be used. For example, $K_2CO_3$ converts an OH group into $O\text{-}M^+$ to promote nucleophilic reaction of an $O^-$ group with a halogenated alkyl ($X\text{-}R_1$, X: halogen, $R_1$: alkyl group).

The alkylation reaction is preferably performed by reaction in an environment of 70°C or more and 100°C or less for about 1 hour. By changing the molar ratio of a luminescent compound having a halogenated alkyl group to a polyphenol, the proportion of the luminescent group introduced to the polyphenol can be adjusted at an intended value.

In the step of chemically modifying a polyphenol with a luminescent group, Michael addition reaction may be

performed.

In the step of chemically modifying a polyphenol with a chain hydrocarbon group, for example, esterification is performed. Specifically, a polyphenol is reacted with an alkyl carboxylic acid in a solvent such as dimethylformamide and dimethyl sulfoxide in the presence of an acidic catalyst, and the polyphenol can be chemically modified with the alkyl carboxylic acid as a chain hydrocarbon group.

As the acidic catalyst, a catalyst donating $H^+$, such as concentrated sulfuric acid, phosphoric acid, and toluenesulfonic acid, can be used.

The esterification is preferably performed by reaction in an environment of 20°C or more and 50°C or less for about 24 hours. By changing the molar ratio of an alkyl carboxylic acid to a polyphenol, the proportion of the alkyl group introduced to the polyphenol can be adjusted at an intended value.

In the step of chemically modifying a polyphenol with a chain hydrocarbon group, for example, Williamson ether synthesis, an alkylation reaction, may be performed. Specifically, a polyphenol can be chemically modified with a halogenated alkyl as the chain hydrocarbon group in a solvent such as tetrahydrofuran and dimethyl sulfoxide in the presence of a basic catalyst.

As the basic catalyst, one or two or more catalysts selected from the group of MH, $M_2CO_3$, and M (M: alkali metal) can be used. For example, $K_2CO_3$ converts an OH group into $O\text{-}M^+$ to promote nucleophilic reaction of an $O^-$ group with a halogenated alkyl ($X\text{-}R_1$, X: halogen, $R_1$: alkyl group).

The alkylation reaction is preferably performed by reaction in an environment of 70°C or more and 100°C or less for about 1 hour. By changing the molar ratio of a halogenated alkyl to a polyphenol, the proportion of the alkyl group introduced to the polyphenol can be adjusted at an intended value.

In the step of chemically modifying a polyphenol with a chain hydrocarbon group, a material having a leaving group such as a sulfonyl group may be used in place of the halogenated alkyl. An alkylation reaction other than the Williamson ether synthesis may be performed. Dehydration-condensation reaction with a carboxylic acid by using a condensing agent such as N,N'-dicyclohexylcarbodiimide (DCC) or condensation reaction with an isocyanate may also be performed.

<Effects of first embodiment>

The above polyphenol derivative pertaining to the first embodiment has the following effects.

(1) The polyphenol derivative contains a polyphenol that is a plant-derived antibacterial or antiviral component and is free from metal such as silver as an antibacterial or antiviral substance.
Accordingly, the polyphenol derivative pertaining to the present embodiment suppresses coloring caused by metal such as silver and suppresses an increase in production cost. The polyphenol derivative pertaining to the present embodiment contains a polyphenol that is a plant-derived antibacterial or antiviral component and thus is highly safe.

(2) In the polyphenol derivative, hydrogen atoms of some hydroxy groups or some hydrogen atoms of aromatic rings of a polyphenol are substituted by a luminescent group.
Accordingly, the polyphenol derivative emits light from the luminescent group bonded to a polyphenol where the polyphenol exists, and enables visual identification of the antibacterial or antiviral effect arising from the polyphenol.

(3) In the polyphenol derivative, hydrogen atoms of some hydroxy groups or some hydrogen atoms of aromatic rings of a polyphenol may be substituted by a chain hydrocarbon group.
Accordingly, the polyphenol derivative has higher affinity with organic solvents, and thus the polyphenol derivative can be dispersed in an organic solvent to impart antibacterial or antiviral effect onto the surface of a coated face.

2. Second embodiment (polymer material)

A polymer material pertaining to a second embodiment will next be described. The polymer material pertaining to the present embodiment is used as an antibacterial or antiviral agent for imparting antibacterial or antiviral function to the surface of an intended part and enables visual identification of the presence of antibacterial or antiviral function. In addition, the polymer material has higher solvent resistance than the polyphenol derivative pertaining to the first embodiment.

The polymer material pertaining to the present embodiment differs from the polyphenol derivative described in the first embodiment in that the polyphenol derivative is network polymerized to improve the solvent resistance.

<Polyphenol derivative>

A polyphenol used in the present embodiment is substantially the same as the polyphenol described in the first embodiment, is, for example, the tannic acid represented by Chemical Formula (1), and thus is not described.

In the polyphenol derivative in the embodiment, as with the polyphenol derivative in the first embodiment, hydrogen atoms of some hydroxy groups or some hydrogen atoms of aromatic rings of a polyphenol are substituted by a luminescent group. Accordingly, the luminescent group bonded to the polyphenol emits light where the polyphenol exists, and this enables visual identification of the antibacterial or antiviral effect arising from the polyphenol.

In the polyphenol derivative in the embodiment, a plurality of polyphenols are connected by esterification with a bifunctional alkyl dicarboxylic acid, etherification with a bifunctional halogenated alkyl, urethanization with a bifunctional alkyl diisocyanate, or the like, and the polyphenol is network polymerized. Esterification and etherification may be performed by substantially the same method as the synthetic method described in the first embodiment. Accordingly, the hydroxy groups of a polyphenol are reduced to improve the solvent resistance of the polymer material.

In the polyphenol derivative used in the present embodiment, as with the polyphenol derivative in the first embodiment, hydrogen atoms of some hydroxy groups or some hydrogen atoms of aromatic rings of a polymer-networked polyphenol may be substituted by a chain hydrocarbon group.

<Effects of second embodiment>

The above polymer material pertaining to the second embodiment has substantially the same effects as the polyphenol derivative pertaining to the first embodiment.

The above polyphenol derivative and the polymer material usable as an antibacterial or antiviral agent can be applied to various fields. For example, the polyphenol derivative and the polymer material can be applied to the medical field, the agriculture, forestry, and fisheries field, the cosmetic field, the food processing field, the textile and clothing field, the construction field, the bedding field, the shipping field, the electronics field, the water treatment field, and the like.

Example

The polyphenol derivative and the polymer material pertaining to the present disclosure will next be described with reference to examples, but the present invention is not limited to them.

[Example 1]

A tannic acid (manufactured by FUJIFILM Wako Pure Chemical Corporation, 203-06331) was prepared as a polyphenol. The tannic acid, acetyl chloride, and triethylamine were added to dehydrated acetone and were reacted at room temperature for 20 hours to yield an intermediate compound. Subsequently, 1-pyrenebutyryl chloride and triethylamine were added to the dehydrated acetone containing the intermediate compound, and the whole was reacted at room temperature for 20 hours to yield a polyphenol derivative 1. The number of substituted luminescent groups of the synthesized polyphenol derivative 1 was 1, and the number of substituted alkyl groups was 4. The substituted alkyl group had one carbon atom.

[Example 2]

The above tannic acid and potassium carbonate were added to dimethylformamide (DMF) and were dissolved, and an alkyl iodide (n-decyl iodide) and bromomethylpyrene were further added. The whole was reacted at 80°C for 20 hours to yield a polyphenol derivative 2. The number of substituted luminescent groups of the synthesized polyphenol derivative 2 was 1, and the number of substituted alkyl groups was 9. The substituted alkyl group had 10 carbon atoms.

[Example 3]

The above tannic acid, acryloyl chloride, and triethylamine were added to dehydrated acetone and were reacted at room temperature for 20 hours to yield an intermediate compound. Subsequently, 1-pyrenebutyryl chloride and triethylamine were added to the dehydrated acetone containing the intermediate compound, and the whole was reacted at room temperature for 20 hours to yield a polyphenol derivative 3. The polyphenol derivative 3 is to be network polymerized by ultraviolet irradiation in the presence of a photoinitiator. The number of substituted luminescent groups of the synthesized polyphenol derivative 3 was 1, and the number of substituted alkyl groups was 5. The substituted alkyl group had two carbon atoms.

[Example 4]

The above tannic acid, 1-pyrenebutyryl chloride, and triethylamine were added to dehydrated acetone and were reacted at room temperature for 20 hours to yield a polyphenol derivative 4. The number of substituted luminescent groups of the synthesized polyphenol derivative 4 was 1, and the number of substituted alkyl groups was 0.

[Comparative Example 1]

The above tannic acid was prepared. The number of substituted luminescent groups of the tannic acid was 0, and the number of substituted alkyl groups was 0.

<Evaluation>

(Fluorescence spectrum measurement)

The polyphenol derivatives produced in Examples and the tannic acid in Comparative Example 1 were subjected to fluorescence spectrum measurement by using a spectrofluorometer (FP-6600 manufactured by JASCO Corporation). The fluorescence spectrum measurement was performed with excitation light at a wavelength of 365 nm.

FIG. 1 illustrates overlapped spectra of the polyphenol derivatives in Examples 1 to 4 and the tannic acid in Comparative Example 1.

(Light emission visibility test)

The polyphenol derivatives in Examples 1, 2, and 4 (polyphenol derivative 1, polyphenol derivative 2, and polyphenol derivative 4) and the tannic acid in Comparative Example 1 were each dissolved in methyl ethyl ketone (MEK). The solution was applied by drop casting onto a 5 cm × 5 cm glass substrate and was dried at room temperature for 1 hour to yield a film.

The polyphenol derivative 3 in Example 3 and a photoinitiator (IGM Resins B. V. Omnirad184) were dissolved in methyl ethyl ketone (MEK). The solution was applied by drop casting onto a 5 cm × 5 cm glass substrate, and the substrate was irradiated with ultraviolet light (a wavelength of 264 nm, a power density of 120 W/cm) for 2 seconds to yield a film.

The glass substrate samples each having the coating film formed by the above method in Examples and Comparative Example were irradiated with ultraviolet light at 365 nm by using an ultraviolet light emitting device (SLUV4 manufactured by AS ONE Corporation). On each sample surface in Examples and Comparative Example, light emission was visually tested. A sample from which light emission was observed was evaluated as "o", whereas a sample from which no light emission was observed was evaluated as "×".

(Antibacterial test)

The polyphenol derivatives (polyphenol derivatives 1 to 4) in Examples 1 to 4 and the tannic acid in Comparative Example 1 were each applied by the above method onto a 5 cm × 5 cm glass substrate at a weight of 1.2 mg/cm$^2$ to yield a coating film.

The glass substrate samples in Examples and Comparative Example each having the coating film formed by the above method were subjected to antibacterial test by a method in accordance with JIS Z2801, and antibacterial activity values (against E. coli) were determined.

Table 1 shows the evaluation results.

[Table 1]

| | Substrate | Substance | | | | Solvent | Evaluation | |
|---|---|---|---|---|---|---|---|---|
| | | | The number of substituted luminescent groups | The number of substituted alkyl groups | The carbon number of alkyl group | | Light emission visibility test | Antibacterial test (antibacterial activity value) |
| Ex. 1 | Glass substrate | Polyphenol derivative 1 | 1 | 4 | 1 | Methyl ethyl ketone | ○ | >5.8 |
| Ex. 2 | Glass substrate | Polyphenol derivative 2 | 1 | 9 | 10 | Methyl ethyl ketone | ○ | >5.8 |
| Ex. 3 | Glass substrate | Polyphenol derivative 3 | 1 | 5 | 2 | Methyl ethyl ketone | ○ | >5.8 |
| Ex. 4 | Glass substrate | Polyphenol derivative 4 | 1 | 0 | 0 | Methyl ethyl ketone | ○ | >5.8 |
| Comp. Ex. 1 | Glass substrate | Tannic acid | 0 | 0 | 0 | Methyl ethyl ketone | × | >5.8 |

As shown in Table 1, light emission at a wavelength in a visible range was observed from the polyphenol derivatives having a luminescent group in Examples. In contrast, almost no light emission at a wavelength in a visible range was observed from the tannic acid having no luminescent group in Comparative Example 1.

The coating films formed from the polyphenol derivatives in Examples sufficiently achieved substantially the same antibacterial activity as the tannic acid containing no luminescent group in Comparative Example 1 and were ascertained to be usable, for example, for antibacterial or sterilization purpose. On the coating film formed from each polyphenol derivative in Examples, the presence of the compound having antibacterial activity can be easily identified by ultraviolet irradiation, and thus whether the antibacterial function was imparted was able to be easily ascertained.

In Comparative Example 1 using the tannic acid having no luminescent group, antibacterial activity was sufficiently achieved, but the presence of the compound having antibacterial activity was failed to be identified by light emission.

The scope of the disclosure is not limited to the described exemplary embodiments with reference to drawings, but includes all embodiments that have effects equivalent to those intended by the disclosure. The scope of the disclosure is not limited to the combinations of features of the invention as defined by the claims, but may be defined by any intended combination of specific features of all the disclosed features.

## Claims

1. A polyphenol derivative in which a hydrogen atom of some hydroxy groups of a polyphenol or some hydrogen atoms of an aromatic ring of a polyphenol are substituted by a luminescent group.

2. The polyphenol derivative according to claim 1, wherein
the hydrogen atom of the hydroxy group or the hydrogen atoms of the aromatic ring is substituted by the luminescent group such that the number of the luminescent groups is not less than 1 and not more than a value 1 less than a total of the number of hydrogen atoms of the hydroxy groups and the number of hydrogen atoms of the aromatic rings.

3. The polyphenol derivative according to claim 1 or 2, wherein
the luminescent group is at least one luminescent group selected from the group consisting of a pyrene group, an anthracene group, a phenanthrene group, a benzoxazole group, a flavone group, a carbazole group, and a coumarin group.

4. The polyphenol derivative according to claim 3, wherein
the pyrene group is a luminescent group having at least one skeleton selected from the group consisting of a 4-(1-pyrene)-butyric acid skeleton, a 1-pyrenebutyric acid skeleton, and a 1-(methyl)pyrene skeleton.

5. The polyphenol derivative according to any one of claims 1 to 4, wherein
some hydrogen atoms of the hydroxy group and the aromatic ring of the polyphenol are substituted by a chain hydrocarbon group.

6. The polyphenol derivative according to claim 5, wherein
the chain hydrocarbon group is an alkyl group.

7. The polyphenol derivative according to claim 5 or 6, wherein
the chain hydrocarbon group has 1 or more and 18 or less carbon atoms.

8. The polyphenol derivative according to any one of claims 5 to 7, wherein
thedrogen atomsof the hydroxy group or the aromatic ring is substituted by the chain hydrocarbon group such that a total of the number of the luminescent groups and the number of the chain hydrocarbon groups is not more than a value 1 less than a total of the number of hydrogen atoms of the hydroxy groups and the number of hydrogen atoms of the aromatic rings.

9. The polyphenol derivative according to any one of claims 1 to 8, wherein

   the polyphenol is a tannic acid, and
   the polyphenol derivative is a tannic acid derivative.

10. A polymer material comprising:

a polyphenol derivative in which a hydrogen atom of some hydroxy groups of a polyphenol or some hydrogen atoms of an aromatic ring of a polyphenol are substituted by a luminescent group, wherein
the polyphenol derivative is network polymerized.

FIG. 1

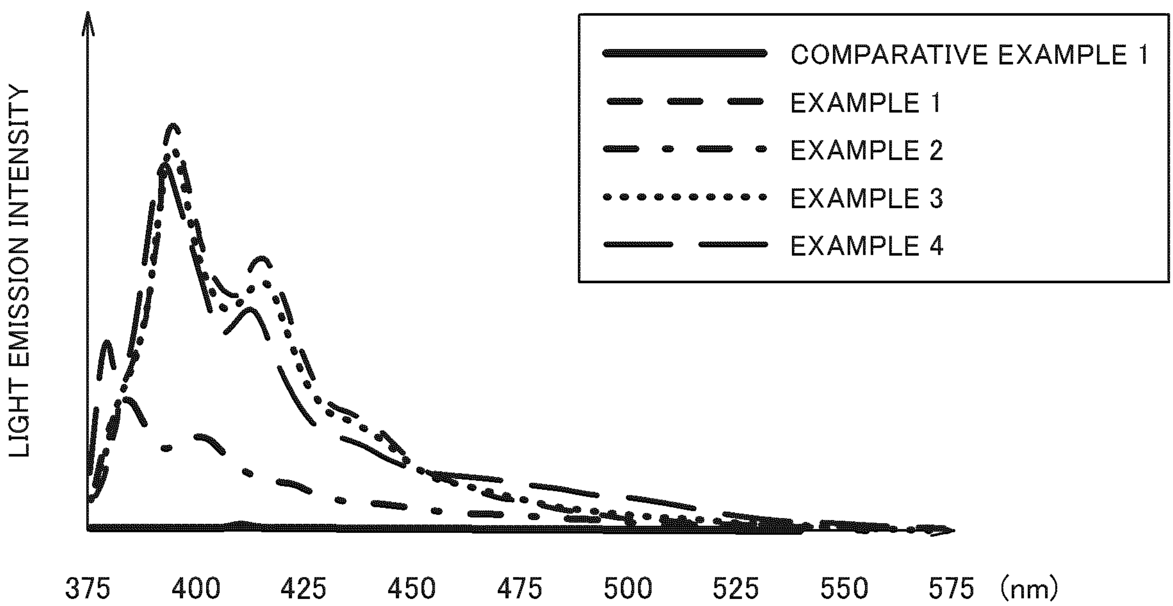
LIGHT EMISSION INTENSITY
COMPARATIVE EXAMPLE 1
EXAMPLE 1
EXAMPLE 2
EXAMPLE 3
EXAMPLE 4
375
400
425
450
475
500
525
550
575
(nm)

## INTERNATIONAL SEARCH REPORT

International application No.
**PCT/JP2021/046806**

**A. CLASSIFICATION OF SUBJECT MATTER**

***C08G 65/40***(2006.01)i; ***C09K 11/06***(2006.01)i; ***A01P 1/00***(2006.01)i; ***A01N 65/00***(2009.01)i; ***A01P 3/00***(2006.01)i
FI: C09K11/06; A01P1/00; A01P3/00; C08G65/40; A01N65/00 G; C09K11/06 610; C09K11/06 630

According to International Patent Classification (IPC) or to both national classification and IPC

**B. FIELDS SEARCHED**

Minimum documentation searched (classification system followed by classification symbols)

C08G65/40; C09K11/06; A01P1/00; A01N65/00; A01P3/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2001-165859 A (LABORATORY OF MOLECULAR BIOPHOTONICS) 22 June 2001 (2001-06-22)<br>claim 1, paragraphs [0009], [0013], [0027], [0056]-[0063], example 7 | 1-4 |
| Y | claim 1, paragraphs [0003]-[0006], [0009], [0013], [0027], [0056]-[0063], [0070], example 7 | 9 |
| Y | paragraph [0013], example 7 | 3-4 |
| Y | JP 2011-42834 A (NISSHIN STEEL CO LTD) 03 March 2011 (2011-03-03)<br>paragraph [0035], fig. 1-2 | 9 |
| Y | JP 2009-268372 A (KIRIN BREWERY CO LTD) 19 November 2009 (2009-11-19)<br>paragraphs [0026]-[0031] | 9 |
| Y | JP 2017-169536 A (SUNTORY HOLDINGS LTD) 28 September 2017 (2017-09-28)<br>paragraphs [0020]-[0021], examples | 9 |
| X | WO 2014/136885 A1 (KONICA MINOLTA, INC) 12 September 2014 (2014-09-12)<br>claim 1, paragraphs [0114]-[0128], [0136], tables 1, 2, examples 32, 36 | 1-2, 10 |
| Y | claim 1, paragraphs [0054], [0060], [0114]-[0128], [0136], tables 1, 2, examples 32, 36 | 3-4 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **28 February 2022** | **08 March 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2021/046806**

**C. DOCUMENTS CONSIDERED TO BE RELEVANT**

| Category* | Citation of document, with indication, where appropriate, of the relevant passages | Relevant to claim No. |
|---|---|---|
| X | JP 2003-238495 A (SANGAKU RENKEI KIKO KYUSHU:KK) 27 August 2003 (2003-08-27)<br>claim 1, paragraphs [0004], [0034]-[0035], [0048]-[0057], examples | 1, 10 |
| A | WO 2016/076311 A1 (NATIONAL INSTITUTE FOR MATERIALS SCIENCE) 19 May 2016 (2016-05-19)<br>entire text, all drawings | 1-10 |
| A | JP 2004-307362 A (WAKAYAMA PREFECTURE) 04 November 2004 (2004-11-04)<br>entire text | 1-10 |
| A | JP 2020-125372 A (NATIONAL INSTITUTE FOR MATERIALS SCIENCE) 20 August 2020 (2020-08-20)<br>entire text, all drawings | 1-10 |
| A | WO 2019/039482 A1 (NATIONAL INSTITUTE FOR MATERIALS SCIENCE) 28 February 2019 (2019-02-28)<br>entire text, all drawings | 1-10 |
| A | WO 2017/204359 A1 (NATIONAL INSTITUTE FOR MATERIALS SCIENCE) 30 November 2017 (2017-11-30)<br>entire text, all drawings | 1-10 |
| A | JP 2008-223111 A (FUKUSHIMA PREFECTURE) 25 September 2008 (2008-09-25)<br>entire text, all drawings | 1-10 |

Form PCT/ISA/210 (second sheet) (January 2015)

**INTERNATIONAL SEARCH REPORT**
**Information on patent family members**

International application No.
**PCT/JP2021/046806**

| Patent document cited in search report | Publication date (day/month/year) | Patent family member(s) | Publication date (day/month/year) |
|---|---|---|---|
| JP 2001-165859 A | 22 June 2001 | (Family: none) | |
| JP 2011-42834 A | 03 March 2011 | (Family: none) | |
| JP 2009-268372 A | 19 November 2009 | (Family: none) | |
| JP 2017-169536 A | 28 September 2017 | (Family: none) | |
| WO 2014/136885 A1 | 12 September 2014 | US 2016/0018300 A1<br>claim 1, paragraphs [0057], [0063], [0145]-[0159], [0167], tables 1, 2, examples 32, 36 | |
| JP 2003-238495 A | 27 August 2003 | (Family: none) | |
| WO 2016/076311 A1 | 19 May 2016 | US 2017/0321061 A1<br>entire text, all drawings | |
| JP 2004-307362 A | 04 November 2004 | (Family: none) | |
| JP 2020-125372 A | 20 August 2020 | (Family: none) | |
| WO 2019/039482 A1 | 28 February 2019 | US 2020/0131398 A1<br>entire text, all drawings | |
| WO 2017/204359 A1 | 30 November 2017 | US 2020/0024447 A1<br>entire text, all drawings | |
| JP 2008-223111 A | 25 September 2008 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015198890 A